# EUROPEAN PATENT SPECIFICATION

(11) **EP 3 884 955 B1**
(45) Date of publication and mention of the grant of the patent: **22.01.2025**
(21) Application number: 21165097.3
(22) Date of filing: 26.03.2021
(51) Int. Cl.: A61K 36/87, A61K 31/05, A61K 31/07, A61K 31/192, A61K 31/352, A61K 31/353, A61K 31/525, A61K 31/713, A61K 33/30, A61P 27/00, A23L 33/105, A61K 9/48

(54) **DIETARY SUPPLEMENT TO COUNTERACT AGE-RELATED MACULAR DEGENERATION**
NAHRUNGSERGÄNZUNGSMITTEL GEGEN ALTERSBEDINGTE MAKULADEGENERATION
COMPLÉMENT ALIMENTAIRE POUR LUTTER CONTRE LA DÉGÉNÉRESCENCE MACULAIRE LIÉE À L'ÂGE

(30) Priority: 27.03.2020 IT 202000006493
(43) Date of publication of application: 29.09.2021
(60) Divisional application: 24213632.3
(73) Proprietor: NGN Healthcare - New Generation Nutraceuticals S.r.l., 83013 Mercogliano (IT)
(72) Inventor: NOVELLINO, Ettore, 83100 Avellino (IT); TENORE, Gian Carlo, 80128 Napoli (IT)
(74) Representative: Valenza, Silvia

(56) References cited:
- US-A1- 2013 303 603
- US-B2- 9 421 189
- MIKA REINISALO ET AL: "Polyphenol Stilbenes: Molecular Mechanisms of Defence against Oxidative Stress and Aging-Related Diseases", OXIDATIVE MEDICINE AND CELLULAR LONGEVITY, vol. 2015, 1 January 2015 (2015-01-01), US, pages 1 - 24, XP055619433, ISSN: 1942-0900, DOI: 10.1155/2015/340520
- TOLUN AYSU ET AL: "Effect of different microencapsulating materials and relative humidities on storage stability of microencapsulated grape pomace extract", FOOD CHEMISTRY, ELSEVIER LTD, NL, vol. 302, 12 August 2019 (2019-08-12), XP085770610, ISSN: 0308-8146, [retrieved on 20190812], DOI: 10.1016/J.FOODCHEM.2019.125347
- MORENO T ET AL: "Storage stability and simulated gastrointestinal release of spray dried grape marc phenolics", FOOD AND BIOPRODUCTS PROCESSING, INSTITUTION OF CHEMICAL ENGINEERS, RUGBY, GB, vol. 112, 5 September 2018 (2018-09-05), pages 96 - 107, XP085528537, ISSN: 0960-3085, DOI: 10.1016/J.FBP.2018.08.011

## Description

### FIELD OF THE INVENTION

The present invention relates to the field of dietary supplements, in particulardietary supplements suitable for maintaining visual function or counteracting age-related macular degeneration.

### BACKGROUND

The expression "macular degeneration" indicates a retinal disease that causes an alteration, a reduction in the functionality of the central area of the retina (the macula) up to a loss of the central vision. It causes a significant and irreversible reduction in visualfunction at the central visual field level. The most common related phenomenon is the aging process of the eye: the macula, which contains numerous photoreceptors, alters to the point of losing its characteristics. This is due to retinal cell death, which can be slow and progressive, or more rapid and dramatic. Age-related macular degeneration (AMD), in addition to being an important cause of low vision, is currently considered the leading cause of central blindness in the most affluent countries and the third one overall. Indicatively, 5% of the world's blindness is attributable to AMD, but this figure rises to 41% in the most affluent countries. It is predicted that, by 2020, approximately 196 million people will be affected by age-related macular degeneration, a figure that is likely to rise with the world's ageing population (especially in the most affluent countries).

The incidence of AMD is rare before the age of 55, but mostly increases after the age of 75. The most serious form of the disease, called "wet", is less frequent and more rapidly evolving, but, at present, it is the only one considered as treatable. Initial symptoms consist of image distortion in the centre of the visual field; difficulty in reading and performing close-range activities where vision of small details is required; loss of colour brilliance. AMD therefore involves a severe penalization, but it should be emphasized that it (even in the most severe cases) does not cause total blindness, as paracentral and lateral vision is preserved. However, it is a highly invalidating pathology, which can also have serious psychological repercussions.

The aetiology of AMD has not yet been demonstrated, but several risk factors associated with its occurrence have been highlighted, like the following ones: age over 55 years, male sex, cigarette smoking, alcohol abuse, diabetes mellitus, sedentary lifestyle, vitamin-poor diet, high blood pressure, coagulation disorders, prolonged and repeated exposure to very intense light sources. In addition, familiarity has been ascertained as the main risk factor in the development of the disease by subjects with first-degree relatives who are affected by it (the origin is in fact genetic).

Dry forms are still considered as incurable; however, it may be possible, once they are diagnosed, to at least partially slow down their evolution (e.g. through a correct lifestyle ranging from regular exercise to a varied diet), although the issue remains scientifically controversial. In addition, there are some cases in which dry forms evolve into wet ones. The wet form (exudative) can be treated with photodynamic therapy, carried out by means of a special type of laser, after an intravenous injection of verteporphyrin which, once activated by the laser light, allows the selective occlusion of the new vessels (it creates thrombi that close off the harmful capillaries), without damaging the surrounding retinal tissue. However, it can be successfully performed only in subfoveal forms, i.e. below the fovea (the central avascular region of the macula) and in iuxtafoveal forms (200-500 microns from the fovea). Repeated sessions over time are often necessary and, unfortunately, sometimes the disease can recur months later (relapse).

The other therapeutic possibility in the exudative forms is represented by intravitreal injections of anti-VEGF drugs. These are substances that act by inhibiting the proliferation of new blood vessels in the retina (antiangiogenic), wh ich causes the appearance of subretinal membranes and bleeding. These drugs (bevacizumab, ranibizumab, aflibercept and pegaptanib sodium are the names of the active ingredients) therefore make it possibleto obtain results in the treatment of exudative macular degeneration with non-scarring neovascular membranes (they can be administered in the case of the wet form, which has a more rapid course but less common than the dry form). Their administration must be carried out in a sterile environment, with all the hygiene standards typical of an operating theatre. A strong slowdown in the evolution of the disease can be achieved. However, because the treatment, to be effective, must be repeated for several months, patients should be monitored for ocular hypertension and those who develop it later should be monitored periodically for changes related to glaucoma.

Given the difficulties of the therapeutic approach to AMD pathology, consisting of a relatively low success rate, complex and often painful applications, and sometimes serious side effects, modern medicine is increasingly oriented to alternative practices, to support or replace the classic drug therapy. Natural antioxidant substances, especially from food sources, could help combat free radical formation (oxidative stress and inflammation playa critical role in the initiation and progression of age-related eye diseases) and ischemia of macular retinal tissue (i.e., its death due to reduced or stopped blood supply to the retina). Natural substances that are officially recognised as having a positive effect on visual ability include vitamins A and B2 and zinc. Today, the European Regulation No. 432/2012 attributes to them the health claim according to which these substances can contribute to the maintenance of normal visual ability.

On the other hand, among the natural antioxidant substances being tested in the ophthalmic field, polyphenols play a particularly interesting role. Specifically, these are the polyphenols naturally present in grapes, the main biologically active components in red wine. In vitro and in vivo (animal model) experimental studies, available in the literature, have provided evidence of the biological effects of polyphenols on numerous oxidative pathways that are implicated in the pathogenesis of these age-related eye disorders. Pre-clinical testing has already corroborated positive effects of polyphenols against AMD, glaucoma, cataracts, and diabetic retinopathy. However, a serious limitation to the effectiveness of polyphenols in the therapeutic field is related to their poor bioavailability. In fact, these are molecules that are very poorly absorbed in the intestine, due to their high polarity and susceptibility to be attacked by oxidasic enzymes, and rapidly metabolized at the systemic level.

Mika Reinisalo et al ("Polyphenol Stilbenes: Molecular Mechanisms of Defence against Oxidative Stress and Aging-Related Diseases", OXIDATIVE MEDICINE AND CELLULAR LONGEVITY, vol. 2015, 1 January 2015, pages 1-24) discloses that polyphenols such as in grape pomaces display a potential for the prevention of retinal diseases such as AMD (age-related macular degeneration).

US 2013/303603 A1 claims muscadine pomace extract and its use in an anti-aging supplement. The extract can be fortified with one or more vitamins and/or minerals including iron, zinc, copper, vitamin A, vitamin C, vitamin B2. Tolun Aysu et al ("Effect of different microencapsulating materials and relative humidities on storage stability of microencapsulated grape pomace extract", FOOD CHEMISTRY, ELSEVIER LTD, NL, vol. 302, 12 August 2019) shows the prolongation of the storage stability of polyphenols, obtained from grape pomace, using a spray drying-based microencapsulation technique.

The object of the present invention is to provide a nutraceutical composition that may be at least a useful alternative to help maintain normal visual ability and/or to counteract age-related macular degeneration.

### DEFINITIONS AND ABBREVIATIONS

AMD: age-related macular degeneration
MaGPE: Maltdextrinated Grape Pomace Extract

### SUMMARY OF THE INVENTION

It has been surprisingly highlighted that it is possible to increase the bioavailability of polyphenols, through microencapsulation in maltodextrins, of the extractive phytocomplex that contains them. Thus, the subject-matter of the present patent application is a gastro-resistant pharmaceutical/nutraceutical composition comprising a grape pomace extract, rich in polyphenols, microencapsulated in maltodextrins (MaGPE), vitamin A, vitamin B2, and zinc.

The nutraceutical composition subject-matter of the present invention is useful in contributing to the maintenance of normal visual ability and/or to counteracting age-related macular degeneration.

It is also subject-matter of the present disclosure to provide a method for preparing a grape pomace extract, microencapsulated in maltodextrins (MaGPE), having high bioavailability of polyphenols, comprising:
i) grape pomace recovery after the winemaking process;
ii) pomace extraction with water at 45-55°C;
iii) centrifugation of the extractive solution;
iv) drying of the extractive solution by means of spray-drying technique, in co-presence of maltodextrins.

### DETAILED DESCRIPTION OF THE INVENTION

For the purposes of the present invention grape pomace extract means a hot aqueous extract at a temperature of 45-50°C. Since the extraction solvent is water, where polyphenols are poorly soluble, it is necessary to perform the extraction at a temperature higher than room temperature in order to increase the solubility of polyphenols in water. However, it is not recommended exceeding 55°C to avoid degradation of the polyphenols.

For the purposes of the present invention, the pomace from which to make the extract can be obtained by recovering the pomace from any winemaking process of any cultivar, preferably from Aglianico cultivar. The pomace extract according to the present invention contains Resveratrol, Gallic Acid, Catechin, Ferulic Acid, Quercetin, Procyanidin B2. The extract of the pomaces of the Aglianico cultivar has a peculiar profile in polyphenols for which 100 mg of dry extract contain:
Resveratrol 0.5-3 mcg;
Gallic acid 100-150 mcg;
Catechin 450-550 mcg;
Ferulic acid 1.5-3 mcg;
Quercetin 2-4.5 mcg;
Procyanidin B2 40-50 mcg;

Preferably, the extract of the present invention is a pomace extract of the Aglianico cultivar, rich in polyphenols, microencapsulated in maltodextrins (Aglianico MaGPE).

The nutraceutical composition of the present invention also comprises vitamin A, vitamin B2 and zinc.

Surprisingly, it has been found that the combination of these components produces a synergistic effect in helping to maintain normal visual ability or in counteracting age-related macular degeneration.

Preferably, vitamin B2 is present as riboflavin or sodium-riboflavin-5'-phosphate; preferably, vitamin A is present as retinol or retinyl acetate or retinyl palmitate or beta-carotene; preferably, zinc is present as zinc acetate or zinc chloride or zinc citrate or zinc gluconate or zinc lactate or zinc oxide or zinc carbonate or zinc sulphate or zinc sulphate.

Preferably, a composition according to the invention comprises or consists of:

| | |
|---|---|
| MaGPE | 75-80% |
| Vitamin A | 0.1 - 0.3% |
| Vitamin B2 | 0.2 - 0.7% |
| Zinc | 2 - 5% |

Excipients (silicon dioxide, corn starch, microcrystalline cellulose, calcium phosphate, talc, aluminum silicate) q.s.
wherein the percentages are in weight calculated with respect to the total weight of the composition.

Preferably, a composition according to the invention comprises or consists of:

| | |
|---|---|
| Aglianico MaGPE | 75 - 80% |
| Vitamin A | 0.1 - 0.3% |
| Vitamin B2 | 0.2 - 0.7% |
| Zinc | 2 - 5% |

Excipients (silicon dioxide, corn starch, microcrystalline cellulose, calcium phosphate, talc, aluminum silicate) q.s.
wherein the percentages are in weight calculated with respect to the total weight of the composition.

The composition, according to the invention, is obtained by simply mixing the components in the required amounts with ordinary mixers.

The composition will normally be formulated in powders, capsules, tablets, using known techniques (encapsulation, compression, controlled release, microgranules, nanocapsules, multilayer) as described in the pharmacopoeia for the preparation of gastro-resistant pharmaceutical formulations for oral use.

A particular example of a pharmaceutical formulation according to the invention comprises gastro-resistant capsules, containing:
280 - 350 mg of Aglianico MaGPE, preferably 300 mg;
0.4 - 1.2 mg vitamin A, preferably 0.8 mg;
0.8 - 2.8 mg vitamin B2, preferably 1.6 mg;
8 - 20 mg zinc, preferably 15 mg.

In a particularly preferred manner, the composition may contain:
Resveratrol 2-6 mcg, preferably 4 mcg;
Gallic acid 330-430 mcg, preferably 390 mcg;
Catechin 1370-1625, preferably 1500 mcg;
Ferulic acid 5-8, preferably 6.5 mcg;
Quercetin 7-13, preferably 10 mcg;
Procyanidin B2 120-140, preferably 130 mcg;
vitamin A 0.4 - 1.2 mg, preferably 0.8 mg;
vitamin B2 0.8 - 2.8 mg, preferably 1.6 mg;
zinc 8 - 20 mg, preferably 15 mg.

A particular example of a pharmaceutical formulation according to the invention is a gastro-resistant capsule and/or tablet comprising:
Aglianico MaGPE, 300 mg; vitamin A, 0.8 mg; vitamin B2, 1.6 g; zinc, 15 mg; capsule constituents (titanium dioxide 1.9%; gellan gum 5.0%; hypromellose q.b. at 100%). In particular, the composition preferably comprises: Resveratrol, 4 mcg; Gallic acid, 390 mcg; Catechin, 1500 mcg; Ferulicacid, 6.5 mcg; Quercetin, 10 mcg; Procyanidin B2, 130 mcg.

Further disclosed is a method for preparing a grape pomace extract, microencapsulated in maltodextrins, having high bioavailability of polyphenols, comprising:
i) grape pomace recovery after any winemaking process;
ii) pomace extraction with water at 45-55°C;
iii) centrifugation of the extractive solution;
iv) drying of the extractive solution by means of spray-drying technique, in co-presence of maltodextrins.

Preferably, the pomaces used in the method of the invention are those recovered from the winemaking process of the Aglianico cultivar.

Preferably the extraction (ii) takes place for a time of 160-200 min, more preferably 180 min.

Preferably, centrifugation (iii) is carried out by using a centrifuge of the Macfuge260 type, operating at 9600 rpm continuously.

Preferably, drying (iv) is carried out by spraying an aqueous solution of maltodextrins at a concentration of 40-70%.

The present invention may be better understood in the lightof the following example embodiments.

### EXPERIMENTAL PART

### EXAMPLE 1 - Preparation of MaGPE

The pomaces of the Aglianico cultivar are recovered after the winemaking process and extracted with water at +50 °C. The extraction solution is centrifuged with the Macfuge 260 system, operating at 9600 rpm continuously. The extraction solution is dried by spray-drying technique, using a Pilotech YC-500 system, in co-presence of maltodextrins at a concentration of 40-70%.

### EXAMPLE 2 - Preparation of gastro-resistant capsules

500 mg gastro-resistant capsules were chosen, the heads and bodies of which consisted of titanium dioxide 1.9%, gellan gum 5.0%, hypromellose q.b. at 100%. A mixture of MaGPE powder, vitamin A, vitamin B2 and zinc, in suitable proportions, was set up to fill the capsules so as to ensure an overall content thus constituted: MaGPE, 300 mg; vitamin A, 0.8 mg; vitamin B2, 1.6; zinc, 15 mg. In particular, the composition preferably comprises: Resveratrol, 4 mcg; Gallic acid, 390 mcg; Catechin, 1500 mcg; Ferulic acid, 6.5 mcg; Quercetin, 10 mcg; Procyanidin B2, 130 mcg.

### EXAMPLE 3 - In vitro data

To verify the duodenal bioaccessibility and bioavailability of polyphenols contained in MaPGE, an experimental protocol capable of simulating gastrointestinal digestion and trans-epithelial permeation was employed. Specifically, an aliquot (5 g) of MaGPE was mixed with 6 mL of artificial saliva consisting of: KCI (89.6 g/L), KSCN (20 g/L), NaH₂PO4 (88.8 g/L), Na₂SO4 (57.0 g/L), NaCl (175.3 g/L), NaHCO₃ (84.7 g/L), urea (25.0 g/L), and 290 mg of α-amylase. The pH of the solution was adjusted to 6.8 with 0.1 M HCl. The mixture was introduced into a plastic bag containing 40 mL of water and homogenized in a Stomacher 80 Microbiomaster (Seward, Worthing, UK) for 3 min. Immediately, 0.5 g of pepsin (14,800 U) dissolved in 0.1 N HCl were added, the pH was adjusted to 2.0 with 6 M HCl, and incubated at 37 °C in a Polymax 1040 orbital shaker (250 rpm) (Heidolph, Schwabach, Germany) for 2 hrs. After gastric digestion, pancreatic digestion was simulated as follows: the pH was raised to 6.5 with 0.5 M NaHCO₃ and then, 5 mL of a mixture of pancreatin (8.0 mg/mL) and bile salts (50.0 mg/mL) (1:1; v/v) dissolved in 20 mL of water were added and incubated at 37 °C in an orbital shaker (250 rpm) for 2 hrs. The digested intestinal sample was lyophilized and stored at -80 °C until further analysis. For the assessment of trans-epithelial permeation, the carcinoma cell line of the human colon Caco2 (HTB-37), provided by the American Type Culture Collection (LGC Promochem, Molsheim, France), was chosen. Cells were cultured (17-21 passages) in Medium Dulbecco's Modified Eagle's medium (DMEM) with 4.5 g/L glucose and supplemented with 12.5% fetal calf serum (FCS), 1% non-essential amino acids, 5 mM L-glutamine, 40 U/mL penicillin, 100 µg/mL gentamicin, and 40 µg/mL streptomycin (DMEMc). Cells were maintained at 37 °C in a humidified Co₂/air atmosphere (5:95, v/v) and were subjected to trypsinization every 7 days. Then, the cells were seeded in transwells (3 µm x 24 mm Transwell^{®} inserts) at 6 x 104 cells/cm². The medium (15 mL of DMEM containing 12.5% FCS)was changed every 2 days until the cells reached confluence (7-8 days). The integrity of monolayers (cultured for 14-15 days) was assessed by measuring transepithelial electrical resistance (TEER) using a Millicell-ERS device (Millipore, Zug, Switzerland). Only Caco2 monolayers with TEER higher than 300 Ω x cm² were used for the experiments. The integrity of the monolayers was verified before, during and after the experiment. Then, Caco2 cell monolayers were gently rinsed twice with PBS, the medium was removed from the apical and basal side of the cultures, the transport medium (TM, Hank's balanced salt solution supplemented with 25 mM glucose and 10 mM HEPES) was added to the apical (2 mL) and basolateral (2 mL) compartment, and the pH was adjusted to 6 or 7.4. After 30 min of incubation, the medium on the apical side was replaced with fresh TM containing lyophilized intestinal digest solutions at increasing concentrations (0; 0.5; 1; 2 or 4 mg). After 4 hoursof incubation at37°C, apical and basal solutions were harvested, and aliquots (5 mL) were filtered through 0.45 µm Phenex-PVDF 17 mm syringe filters (Phenomenex, Torrance, CA). The main polyphenols contained both in the intestinal digest and in the apical and basal solutions were monitored by a HPLC-diode-array detector (DAD) analysis, following the method described by Giusti et al. [Giusti et al., 2017] and Silva et al. [Silva et al., 2012].

The following data were obtained:
duodenal bioaccessibility (%): 50%;
bioavailability (%): 15%.

The experimental data indicate that MaGPE, in gastro-resistant capsules, is able to ensure a duodenal bioaccessibility and bioavailability of the polyphenols, equivalent to those obtainable with an aliquot equal to about 8 L of wine that can be obtained from the same cultivar of Aglianico grapes. These data support the effectiveness of the gastro-resistance and the technique of microencapsulation in maltodextrins of the extract from Aglianico pomace, in increasing, respectively, the duodenal bioaccessibility and bioavailability of the polyphenols contained in MaGPE.

### EXAMPLE 4 - Clinical data

The efficacy of a formulation in gastro-resistant capsules was assessed (based on MaGPE, 300 mg; vitamin A, 0.8 mg; vitamin B2, 1.6 g; zinc, 15 mg; specifically, the composition included: Resveratrol, 4 mcg; Gallic acid, 390 mcg; Catechin, 1500 mcg; Ferulic acid, 6.5 mcg; Quercetin, 10 mcg; Procyanidin B2, 130 mcg) in counteracting age-related macular degeneration in individuals with diagnosedAMD. A monocentric, randomized, parallel-group, placebo-controlled trial was conducted on 60 healthy volunteers, 35 women and 25 women, aged between 55 and 80, of white race. Exclusion criteriawere: smoking, obesity (BMI >30 kg/m²), liver disease, kidney disease, heart disease, intake of drugs and supplements for AMD, intake of drugs and supplements based on resveratrol, grape polyphenols, carotenoids, intense physical exercise (>10 h/week), pregnant women, women suspected of being pregnant, women with planned pregnancy, breastfeeding women, pollen allergy. The subjects received written and oral information regarding the trial protocol before they gave their written consent.

The individuals were randomized into 6 groups. Fourgroups took 2 gastro-resistant capsules per day, containing only one of the components of the formulation for each group; one group took 2 gastro-resistant capsules perday, containing the mix of the components, as per the formulation described above; an additional group took 2 gastro-resistant capsules per day, containing only maltodextrins as a placebo.

The subdivision of treatment into the 6 groups was as follows:
group 1, MaGPE (600 mg/day, amounting to: Resveratrol, 4 mcg/day; Gallic acid, 390 mcg/day; Catechin, 1500 mcg/day; Ferulic acid, 6.5 mcg/day; Quercetin, 10 mcg/day; Procyanidin B2, 130 mcg/day);
group2, vitamin A (1.6 mg/day);
group 3, vitamin B2 (3.2 mg/day);
group 4, zinc (30 mg/day);
group 5, MaGPE (600 mg/day, amounting to: Resveratrol, 4 mcg/day; Gallic acid, 390 mcg/day; Catechin, 1500 mcg/day; Ferulic acid, 6.5 mcg/day; Quercetin, 10 mcg/day; Procyanidin B2, 130 mcg/day) + vitamin A (1.6 mg/day) + vitamin B2 (3.2 mg/day) + zinc (30 mg/day);
group 6, placebo (maltodextrin, 1000 mg/day).

The intervention period lasted 24 weeks. At both time T0 (start of the treatment) and time T6 (end of 24^{th} week), central foveal thickness (CFT) was measured for each patient by means of OCT technique (Computed Optical Tomography).

The results obtained were as follows:

| **Tab. 1. Change in thickness (µm) of the central fovea (CFT) after 36 weeks of treatment.** | | | |
|---|---|---|---|
| **Treatment** | **Pre-treatment** | **Post-treatment** | **Variation (%)** |
| Placebo | 473 ± 118 | 469 ± 107 | -0.8 |
| MaGPE | 425 ± 110 | 405 ± 109 | -4.6 |
| Vitamin A | 486 ± 121 | 463 ± 125 | -4.8 |
| Vitamin B2 | 398 ± 99 | 380 ± 96 | -4.4 |
| Zinc | 406 ± 105 | 396 ± 94 | -2.5 |
| Mix | 383 ± 102 | 279 ± 89 | -23.8 |
| The values are expressed as mean ± SD (n = 5). | | | |
| The results were significantly different at a level P = 0.001. | | | |

As can be seen, the formulation called mix, comprising MaGPE, vitamin A, vitamin B2 and zinc, promoted the greatest decrease in the thickness of the central fovea, greater than the sum of the effects of the components taken individually. Such formulation clearly indicates a synergistic effect between the various constituents of the formulation.

The results obtained indicate the possibility of an innovative treatment that can represent a valid alternative in clinical practice.

### References

Giusti, F.; Caprioli, G.; Ricciutelli, M.; Vittori, S.; Sagratini, G. Determination of fourteen polyphenols inpulses by high performance liquid chromatography-diode array detection (HPLC-DAD) and correlation study with antioxidant activity and colour. Food Chem. 2017, 221, 689-697.
Silva, M.A.; Ky, I.; Jourdes, M.; Teissedre, P.L. Rapid and simple method for the quantification of flavan-3-ols in wine. Eur. Food Res. Technol. 2012, 234, 361-365.

## Claims

1. A gastro-resistant pharmaceutical/nutraceutical composition comprising a grape pomace extract, rich in polyphenols, microencapsulated in maltodextrin (Maltodextrinated Grape Pomace Extract: MaGPE), vitamin A, vitamin B2 and zinc.

2. The composition according to claim 1 wherein the grape pomace extract is obtained by extraction with water at a temperature of 45-55 °C.

3. The extract according to any of claims 1-2 in which the pomace is from the Aglianico cultivar.

4. The composition according to any one of claims 1-3 wherein the grape pomace extract comprises Resveratrol, Gallic acid, Catechin, Ferulic acid, Quercetin, Procyanidin B2.

5. The composition according to anyone of claims 1-4 wherein vitamin B2 is present as riboflavin or sodium-riboflavin-5'-phosphate; vitamin A is present as retinol or retinyl acetate or retinyl palmitate or beta-carotene; zinc is present as zinc acetate or zinc chloride or zinc citrate or zinc gluconate or zinc lactate or zinc oxide or zinc carbonate or zinc sulphate.

6. The composition according to any of claims 1-5 comprising or consisting of
| | |
|---|---|
| MaGPE | 75-80% |
| Vitamin A | 0.1-0.3% |
| Vitamin B2 | 0.2-0.7% |
| Zinc | 2-5% |
| Excipients | q.s. |
wherein the percentages are in weight calculated with respect to the total weight of the composition.

7. The composition according to any of claims 1-6 for use in counteracting age-related macular degeneration and maintaining normal visual ability.

## Patentansprüche

1. Magensaftresistente pharmazeutische/nutrazeutische Zusammensetzung, die Traubentresterextrakt, der reich an Polyphenolen ist und in Maltodextrin mikroverkapselt ist (Maltodextrinated Grape Pomace Extract/maltodextrinierter Traubentresterextrakt: MaGPE), Vitamin A, Vitamin B2 und Zink umfasst.

2. Zusammensetzung nach Anspruch 1, wobei der Traubentresterextrakt durch Extraktion mit Wasser bei einer Temperatur von 45-55 °C erhalten wird.

3. Extrakt nach einem der Ansprüche 1-2, wobei die Trester von Aglianico cultivar stammen.

4. Zusammensetzung nach einem der Ansprüche 1-3, wobei der Traubentresterextrakt Resveratrol, Gallussäure, Catechin, Ferulasäure, Quercetin, Procyanidin B2 umfasst.

5. Zusammensetzung nach einem der Ansprüche 1-4, wobei Vitamin B2 als Riboflavin oder Natriumriboflavin-5'-Phosphat vorliegt; Vitamin A als Retinol oder Retinylacetat oder Retinylpalmitat oder Beta-Carotin vorliegt; Zink als Zinkacetat oder Zinkchlorid oder Zinkcitrat oder Zinkgluconat oder Zinklactat oder Zinkoxid oder Zinkcarbonat oder Zinksulfat vorliegt.

6. Zusammensetzung nach einem der Ansprüche 1-5, umfassend oder bestehend aus
| | |
|---|---|
| MaGPE | 75-80% |
| Vitamin A | 0,1-0,3% |
| Vitamin B2 | 0,2-0,7% |
| Zink | 2-5% |
| Hilfsstoffe | q.s. |
wobei die Prozentangaben in Gewicht sind und bezogen auf das Gesamtgewicht der Zusammensetzung berechnet sind.

7. Zusammensetzung nach einem der Ansprüche 1-6 zur Verwendung gegen altersbedingte Makuladegeneration und zur Aufrechterhaltung der normalen Sehfähigkeit.

## Revendications

1. Composition pharmaceutique/nutraceutique gastro-résistante comprenant un extrait de marc de raisin, riche en polyphénols, microencapsulé dans de la maltodextrine (extrait de marc de raisin maltodextriné : MaGPE), vitamine A, vitamine B2 et zinc.

2. Composition selon la revendication 1, dans laquelle l'extrait de marc de raisin est obtenu par extraction avec de l'eau à une température entre 45 et 55 °C.

3. Extrait selon l'une quelconque des revendications 1 à 2, dans lequel le marc provient du cépage Aglianico.

4. Composition selon l'une quelconque des revendications 1 à 3, dans laquelle l'extrait de marc de raisin comprend du resvératrol, de l'acide gallique, de la catéchine, de l'acide férulique, de la quercétine, de la procyanidine B2.

5. Composition selon l'une quelconque des revendications 1 à 4, dans laquelle la vitamine B2 est présente sous forme de riboflavine ou de riboflavine-5'-phosphate de sodium ; la vitamine A est présente sous forme de rétinol ou d'acétate de rétinyle ou de palmitate de rétinyle ou de bêta-carotène ; le zinc est présent sous forme d'acétate de zinc ou de chlorure de zinc ou de citrate de zinc ou de gluconate de zinc ou de lactate de zinc ou d'oxyde de zinc ou de carbonate de zinc ou de sulfate de zinc.

6. Composition selon l'une quelconque des revendications 1 à 5, comprenant ou consistant en
| | |
|---|---|
| MaGPE | 75 à 80% |
| Vitamine A | 0,1 à 0,3% |
| Vitamine B2 | 0,2 à 0,7% |
| Zinc | 2 à 5% |
| | |
|---|---|
| Excipients | q.s. |
dans laquelle les pourcentages sont en poids, calculées en fonction du poids total de la composition.

7. Composition selon l'une quelconque des revendications 1 à 6, pour utilisation dans la lutte contre la dégénérescence maculaire liée à l'âge et le maintien de la capacité visuelle normale.
